# EUROPEAN PATENT SPECIFICATION

(11) **EP 0 413 383 B1**
(45) Date of publication and mention of the grant of the patent: **18.06.1997**
(21) Application number: 90202117.9
(22) Date of filing: 02.08.1990
(51) Int. Cl.: C12N 15/27, C12P 21/02, A61K 38/00, C12P 21/08

(54) **Mutants of human interleukin-3**
Mutanten von Interleukin-3
Mutants de l'interleukine-3

(30) Priority: 14.08.1989 EP 89202082; 14.09.1989 EP 89202331
(43) Date of publication of application: 20.02.1991
(60) Divisional application: 96203516.8
(73) Proprietor: GIST-BROCADES N.V., NL-2600 MA Delft (NL)
(72) Inventor: Dorssers, Lambertus Christiaan Johannes, NL-6668 An Randwijk (NL); Van Leen, Robert Willem, NL-2914 TH Nieuwerkerk a/d IJssel (NL)
(74) Representative: Visser-Luirink, Gesina, Dr.

(56) References cited:
- EP-A- 0 282 185
- WO-A-88/05649

## Description

### Technical Field

The present invention relates to mutants of colony stimulating factors, obtained by recombinant DNA techniques. Specifically, the invention relates to mutants of interleukin-3. More specifically, said mutants contain one or more deletions. The pharmacological applicability of these mutants depends on their specific receptor binding and signal transducing properties.

### Background of the Invention

Historically, factors affecting hematopoietic cells were detected in an assay measuring the proliferation and/or differentiation of bone marrow cells in soft agar cultures. The factors showing this activity have been collectively called colony-stimulating factors (CSFs). More recently it has been found that a variety of CSFs exist which, in part, can be classified by the hematopoietic lineages that are stimulated.

In human and murine systems, these proteins include G-CSF and M-CSF. These proteins stimulate the in vitro formation of predominantly neutrophilic granulocyte and macrophage colonies, respectively. Interleukin-2 ("IL-2") stimulates the proliferation of both activated T-cells and activated B-cells, but is not considered a colony stimulating factor.

GM-CSF and Interleukin-3 ("IL-3", also known as "Multi-CSF") stimulate the formation of both macrophage and neutrophilic and eosinophilic granulocyte colonies. In addition, IL-3 stimulates the formation of mast, megakaryocyte and pure and mixed erythroid colonies (D. Metcalf, "The hematopoietic colony-stimulating factors", 1984, Elsevier, Amsterdam, and D. Metcalf, Science 299 (1985) 16-22).

Growth factor-induced cell proliferation is a complicated process. Following highly specific binding of the growth factor to its receptor at the cell surface, the complex is internalized by endocytosis and induces an intracellular response, often preceded by phosphorylation of the receptor (Sibley et al., Cell 48 (1987) 913-922). These intracellular signals result in specific gene transcription and finally in DNA synthesis and cell replication.

There is considerable interest in the CSFs, since they have a therapeutic potential which consists of restoring depressed levels of hematopoietic and lymphoid stem cell-derived cells.

Human IL-3 ("hIL-3") is such a CSF. Mature hIL-3 consists of 133 amino acids; the protein contains one disulfide bridge and has two potential glycosylation sites (Yang et al.,Cell 47 (1986) 3-10). It has inter alia the following activities:
1) stimulation of colony formation by human hemopoietic progenitor cells wherein the colonies formed include erythroids, granulocytes, megakaryocytes, granulocyte macrophages, and mixtures thereof;
2) stimulation of DNA synthesis by human acute myelogenous leukemia (AML) blasts.

Useful agonists and antagonists of a protein can be created once the structure-function relationship of the molecule is understood. Generally, this relationship is studied by modifying, replacing or deleting amino acids. In this way information can be obtained about the importance of each of the amino acids for the activity of the protein. Important domains of proteins may be the active site, metal and cofactor binding sites, receptor binding sites, the amino acids involved in subunit interactions and the antigenic determinants.

Once the primary sequence of a protein has been determined, various procedures can be employed to study the above-mentioned characteristics. For example, if primary structures of homologous proteins from other species are available these sequences can be aligned. Conserved sequences are often indicative of the importance of certain amino acids.

Secondary structures can be predicted with the use of known algorithms. See, e.g., Hopp and Woods, Proc. Natl. Acad. Sci. USA 78 (1981) 3824-3828, Garnier et al., J. Mol. Biol. 120 (1978) 97-120, Biou et al., Prot. Eng. 2 (1988) 185-191, Cármenes et al., Biochem. Biophys. Res. Commun. 159 (1989) 687-693.

If interspecies homology between homologous proteins is high and the 3-D structure of one of them is known, important amino acids can also be deduced from this structure.

Primary and/or spatial-structure data can be used to make an educated guess for mutagenesis experiments. Expression of mutagenized proteins and the testing of these muteins in biological assays provides information about the relative importance of certain amino acids.

The aim of the present invention is to provide IL-3 mutants with similar or improved pharmaceutical properties with respect to the native IL-3, preferably using the procedures mentioned above.

### Relevant Literature

### Human Interleukin-3

In 1984, cDNA clones coding for murine IL-3 were isolated (Fung et al., Nature 307 (1984) 233-237 and Yokota et al., Proc. Natl. Acad. Sci. USA 81 (1984) 1070-1074). This cDNA would not hybridize with human DNA or cDNA clones. Thus, it was speculated that a human counterpart for murine IL-3 (mIL-3) did not exist. This belief was reinforced by the wide spectrum of activities of the human GM-CSF. Finally, in 1986 a gibbon cDNA expression library provided the gibbon IL-3 sequence. This sequence was subsequently used as a probe against a human genomic library. This provided evidence for the presence of IL-3 in human beings (Yang et al., Cell 47 (1986) 3-10).

Meanwhile, Dorssers et al., Gene 55 (1987) 115-124, found a clone from a human cDNA library that surprisingly hybridized with mIL-3. This hybridization was the result of the high degree of homology between the 3' noncoding regions of mIL-3 and hIL-3.

### Modified CSFs (other than IL-3)

Moonen et al., Proc. Natl. Acad. Sci. USA 84 (1987) 4428-4431 describe the production of human GM-CSF by several recombinant sources including E. coli, yeast and animal cells. Partially purified expression products from yeast and animal cells were assayed for the effect of deglycosylation. The immunoreactivity was increased 4-to 8-fold upon removal of the N-linked oligosaccharides. The specific biological activity was increased by a factor 20 both in the chronic myelogenous leukemia (CML) and in the human bone marrow assay.

Kaushansky et al., Proc. Natl. Acad. Sci. USA 86 (1989) 1213-1217, tried to define the region(s) of the GM-CSF polypeptide required for biological activity. Since human and murine GM-CSF do not crossreact in their respective colony-forming assays the approach was based on the use of hybrid DNA molecules containing various lengths of the coding regions for h- and mGM-CSF. After expression in COS cells, the hybrid proteins were tested in both human and murine colony-forming assays. Two regions of GM-CSF were found to be critical for hematopoietic function. These regions are structurally characterized by an amphiphilic helix and by a disulfide-bonded loop.

Gough et al., Eur. J. Biochem. 169 (1987) 353-358, describe internal deletion mutants of murine GM-CSF. None of the mutants is reported to show biological activity.

Kuga et al., Biochem. Biophys. Res. Com. 159 (1989) 103-111, describe mutagenesis of human G-CSF. The results indicate that most of the expression products with mutations localized in the internal or C-terminal regions abolish hG-CSF activity. On the other hand N-terminal deletion mutants missing 4, 5, 7 or 11 amino acids, out of a total of 174 amino acids, retained activity. Some of the N-terminal amino acid mutants showed increased activity.

Deletion mutants of human interleukin-1 (IL-1) have been created using available endonuclease restriction sites and expression in eukaryotic cells. The carboxyl terminal third (63 amino acids) of the polypeptide contains the active site (Makino et al., Proc. Natl. Acad. Sci. USA 84 (1987) 7841-7845). A recent study of IL-1α and IL-1β showed that 140 and 147 amino acids respectively (out of a total of 153 amino acids), are required for full biological activity (Mosley et al., Proc. Natl. Acad. Sci. USA 84 (1987) 4572-4576). Single amino acid changes at both termini resulted in a significant decrease of biological activity. However, no detailed information with respect to the receptor-binding domain of IL-1 has been obtained from these studies.

Activity of human interleukin-2 was shown to be severely inhibited by removal of both Cys⁵⁸ and Cys¹⁰⁵, whereas deletion of the third Cys residue (125) had no effect (Wang et al., Science 224 (1984) 1431-1433; Cohen et al., Science 234 (1986) 349-352). All substitutions resulting in a disturbance of helical folding of this protein, were found to give significant reductions of biological activity. The potential receptor binding site of IL-2 has been mapped on an eleven amino acid long peptide. Individual amino acid substitutions in this region had dramatic effects (Cohen et al. (supra); Zurawski and Zurawski, EMBO J. 7 (1988) 1061-1069). Mutational analysis further revealed that different domains of IL-2 are involved in high and low affinity binding of the IL-2 receptor complex (Robb et al., Proc. Natl. Acad. Sci. USA 85 (1988) 5654-5658; Collins et al., Proc. Natl. Acad. Sci. USA 85 (1988) 7709-7713).

It can be concluded that modifications of CSF's generally result in dramatic changes in the biological activity of these molecules, e.g. human G-CSF loses its activity after internal or C-terminal mutations have been introduced. N-terminal deletions in human G-CSF of up to 11 amino acids (out of 174 amino acids) do not adversely affect the activity, the same holds for IL-1 where at least 140 out of 153 amino acids are required for full activity. Clearly, less than 10 % of the amino acids can be missed in these molecules.

### Modified IL-3

Clark-Lewis et al., Science 231 (1986) 134-139, performed a functional analysis of synthetic murine IL-3 analogues. They conclude that the stable tertiary structure of the complete molecule was required for full activity. A study on the role of the disulfide bridges showed that replacement of two of the four Cys residues by Ala (Cys⁷⁹, Cys¹⁴⁰ -> Ala⁷⁹, Ala¹⁴⁰) resulted in an increased activity (Clark-Lewis et al., Proc. Natl. Acad. Sci. U.S.A. 85 (1988) 7897-7901).

Literature on proposed modifications of hIL-3 is scarce. Literature on actually performed modifications of hIL-3 is even more scarce. International Patent Application (PCT) WO 88/00598 discloses a 'naturally occurring' Ser²⁷ -> Pro²⁷ replacement. [It should be noted that the numbering of amino acids in WO 88/00598 includes the signal peptide of 19 amino acids.] Furthermore, suggestions are made to replace Cys by Ser, thereby breaking the disulfide bridge, and to replace one or more amino acids at the glycosylation sites, Asn³⁴Cys³⁵Ser³⁶ and Asn⁸⁹Ala⁹⁰Ser⁹¹.

EP-A-0275598 (WO 88/04691) illustrates that Ala¹ can be deleted while retaining biological activity. Some mutant hIL-3 sequences are provided, viz. two double mutants, Ala¹ -> Asp¹, Trp¹³ -> Arg¹³ (pGB/IL-302) and Ala¹ -> Asp¹, Met³ -> Thr³ (pGB/IL-304) and one triple mutant Ala¹ -> Asp¹, Leu⁹ -> Pro⁹, Trp¹³ -> Arg¹³ (pGB/IL-303) [numbering starts with the first amino acid of the mature protein.]

WO 88/05469 describes how the deglycosylation mutants mentioned above can be obtained and also mutants of Arg⁵⁴Arg⁵⁵ and Arg¹⁰⁸Arg¹⁰⁹Lys¹¹⁰ (converted to the same numbering as in EP-A-0275598). The latter are suggested in order to avoid proteolysis upon expression in Saccharomyces cerevisiae by KEX2 protease. No mutated proteins are disclosed. Glycosylation and the KEX2 protease activity are only important, in this context, upon expression in yeast.

Finally, EP-A-0282185 mentions various mutants that may be conformationally and antigenically neutral. To achieve this a series of synonymous amino acid substitutions are suggested. The proposed amino acid changes are aimed at keeping the structure and charge distributiuon of the IL-3 molecule unaltered. The only actually performed mutations are Met² -> Ile² and Ile¹³¹ -> Leu¹³¹. It is not disclosed whether the contemplated neutralities are obtained.

No known extensive mutagenesis experiments on hIL-3 have been disclosed to date.

The present invention provides two new classes of pharmacologically interesting compounds, viz. deletion mutants and substitution mutants of hIL-3, showing biological activities similar and in some cases antagonistic to those of hIL-3.

### SUMMARY OF THE INVENTION

In one aspect of the invention, biologically active polypeptide analogs of interleukin-3 (also referred to hereinafter as "hIL-3 mutants" or "muteins") are provided having a deletion of at least 2 amino acids.

Preferred mutants are those having one or more deletions at the N-terminus (amino acids 1-14) and/or the C-terminus (amino acids 120-130 and/or 130-133).

The mentioned polypeptides are obtained through expression of suitably modified DNA sequences. Thereto the present invention also provides suitable expression vectors and host cells compatible therewith.

In yet other aspects, the invention comprises pharmaceutical compositions that include biologically active peptide analogs of hIL-3 as described above, in combination with a pharmaceutically acceptable carrier.

These and other aspects will be further outlined in the following detailed description.

### BRIEF DESCRIPTION OF THE DRAWINGS

Figure 1 shows the nucleotide and translated amino acid sequence of the fusion protein insert of pGB/IL-336. Amino acids indicated with lower case letters are non-hIL-3 amino acids, the IL-3 protein sequence starts at Ala 1.

Figure 2 shows the 5' and 3' sequence changes introduced to obtain pGB/IL-339. As in Figure 1 lower case lettered amino acids indicate non-hIL-3 amino acids, the mature IL-3 protein sequence starts at Ala 1.

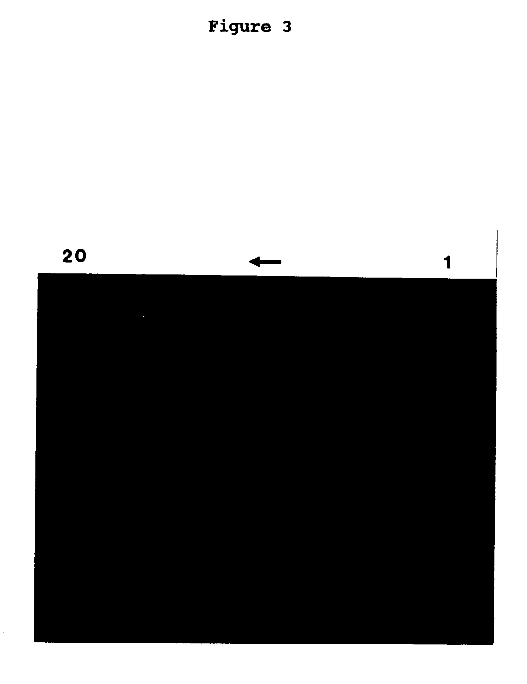

Figure 3 is a photograph of the polyacrylamide gel of the following 19 IL-3 mutants (lanes 1 to 19 respectively): pGB/IL-336, pGB/IL-339, 932, 810, 934, 812, 935, 813, 936, 820, 937, 821, 938, 822, 939, 9329, 904, 905, 9045; and the Pharmacia LMW marker (lane 20). Mutations corresponding with these numbers are given in Table 4.

Figure 4 shows an AML proliferation assay for IL-3 (*) and IL-3 mutant 821 (^{.}).

Figure 5 shows an IL-3-receptor binding experiment. Radiolabeled IL-3 binding to patient AML blasts is competed with unlabeled IL-3 protein (reference preparation). The levels of non-specific (lower bar) and maximal total binding (upper bar) in this experiment are indicated (see Example 5).

Figure 6 shows a comparison of selected mutant IL-3 proteins with respect to relative biological activity (open bars) and relative binding activity (closed bars). The percentage of activity compared to the reference IL-3 preparation is indicated (Table 4).

### DETAILED DESCRIPTION OF THE INVENTION

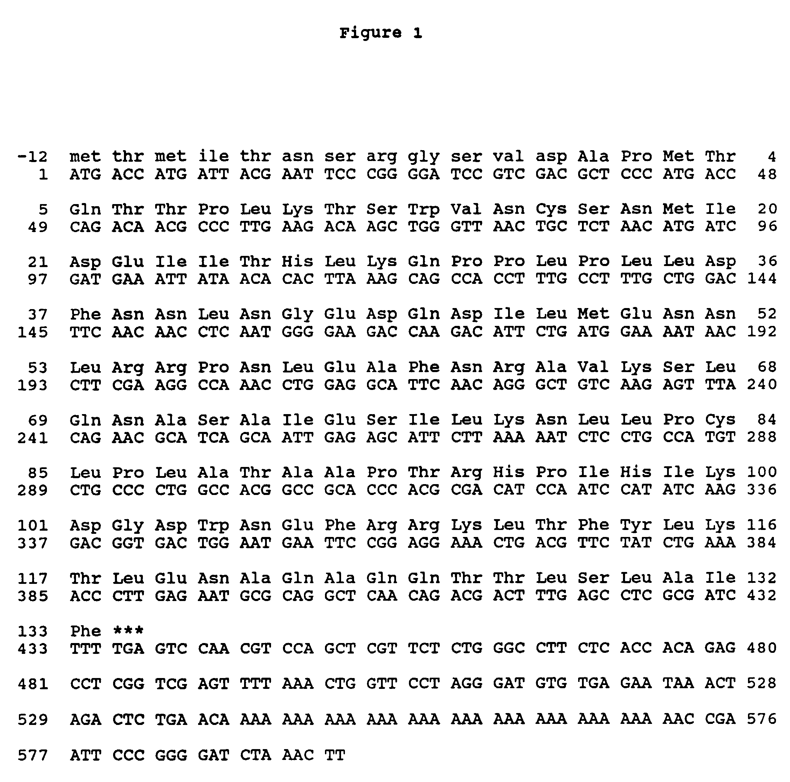

As used herein human IL-3 corresponds to the amino acid (1-133) as depicted in Figure 1.

Human IL-3 is further characterized by its biological activity to stimulate colony formation by human hematopoietic progenitor cells. The colonies formed include erythroids, granulocytes, megakaryocytes, granulocyte macrophages and mixtures thereof. The biological activity of hIL-3 can (for the present purpose) be further devided in two parts; signal transduction and receptor binding.

With an hIL-3 analog, exhibiting improved properties we mean that the analog has a better signal transduction/receptor binding ratio. Where better is dependent on the desired properties. For example, a higher binding coefficient for an antogonist or an equal or higher biological activity for a deletion mutant. Better can also mean that the polypeptide analog is less susceptible to forming aggregates.

For the present purpose the (biological) activity of the human IL-3 analogs is determined by DNA synthesis by human acute myelogenous blasts. Furthermore, receptor binding assays are performed with donor leucocytes, patient AML cells or MV4-11 cells.

IL-3 is active in both receptor binding and signal transduction. If it would be possible to attribute these activities to specific amino acid sequences, which may be either continuous or discontinuous, it would become possible to uncouple the binding and signal transducing activities. Thus enabling the production of specific IL-3 receptor antagonists.

As a first step to achieve these goals a firm understanding of the structure-function relationship of hIL-3 is required. The properties of the naturally occurring or naturally mutated IL-3 may be altered by introducing a variety of mutations in the protein. Such alterations are suitably introduced using the mutagenesis techniques described herein and the mutated molecules (hIL-3 muteins) are suitably synthesized using the expression vectors described. In order to determine the structure-function relationship the following mutagenesis techniques are inter alia to be considered:
a) replacement of one amino acid by an isosteric residue of different function e.g. Asp by Asn. This maintains the hydrogen bonding characteristics, while removing the charge;
b) replacement of one amino acid by another of identical function but different structure e.g. Glu by Asp, where a carboxyl group would be moved by about 1Å;
c) replacement of one amino acid by another of different function;
d) introduction or removal of disulfide bridges;
e) construction of deletion mutants;
f) introduction of helix breakers (e.g. Pro);
g) construction of insertion mutants;
h) generation of glycosylation mutants.

One aspect of the present invention is the development of a specific antagonist which blocks the receptor by binding specifically to it, preferably with a high binding coefficient, thereby hindering the signal transduction.

An antagonist can be any chemical molecule that binds to the receptor and thereby avoids binding of the agonist. The present invention specifically aims at proteins having such a blocking effect. In one of its aspects, the present invention provides hIL-3 muteins which may have at least a partial antagonistic effect. It may be envisaged that administration of a significant dosis of the described mutants will prevent the binding of the physiologically available IL-3, thereby exhibiting its antagonistic activity.

In another aspect of the present invention, there are provided proteins having hIL-3 or hIL-3-like activity, which are smaller, preferably from about 3 to about 25%, than the native hIL-3 molecule. As is further illustrated by the Examples it can be determined by introducing specific deletions which part of the molecule is indispensable for receptor binding and signal transduction.

Deletion mutants have specifically been made. It is to be understood that combinations of these mutants can easily be obtained by the mentioned methods.

Deletion mutants have been made, together covering virtually the complete coding region. Deletions as used herein can be any amino acid deletion of minimally two consecutive (or separated) amino acids. Preferably, four or more amino acids can be deleted. Said deletions can start with any amino acid in the molecule (except of course near the C-terminus). It is also possible to have two separated deletions in the molecule (e.g. two times one amino acid deletion, two times two etc.). In one of the most preferred embodiments 32 amino acids are deleted, 14 at the N-terminus and 18 at the C-terminus of the protein. Surprisingly, all of the expressed deletion mutant proteins showed specific IL-3 biological activity, albeit mostly strongly reduced. Hence, it can be concluded that there is not a single continuous peptide domain on the protein that binds with the receptor.

Four of the mutants, the N-terminal deletion mutant missing amino acids 1 to 14, the C-terminal deletion mutant missing amino acids 120 to 130, the double mutant thereof and the C-terminal mutant 130-133, showed biological and binding activity which is comparable to that of the native IL-3 molecule. It is surprising that hIL-3 missing up to 25 amino acids (about 20% of the full length hIL-3) still retains its full activity. In view of Cys¹⁶ which is involved in the formation of a disulfide bridge it is to be expected that a -15 N-terminal deletion mutant retains its full activity. The double deletion mutants missing the 14 N-terminal and upto 18 C-terminal amino acids (Table 4, X and Z) still retain the full activity. Such mutants, which lack up to 32 amino acids miss at most approximately 25% of the native molecule. Since this double mutant retains full activity it is to be expected that the -18 C-terminal mutant will also be fully active.

Mutants having only internal deletions have also been made. An internal deletion mutant with completely retained activity is described in this invention, this mutant lacks amino acids 120 to 130 (Table 4, 939). It is likely that more such mutants can be made. For processing reasons it may be advantageous to retain the first few amino acids of the mature IL-3 protein. Since the -14 N-terminal deletion mutant retains full activity it is expected that an internal deletion mutant in the N-terminal region will also retain its activity. Another region where a deletion could be introduced successfully seems the Gln²⁹ - Leu³⁵ region. Deletion of 1 to 7 amino acids, possibly in combination with a Lys²⁸ -> Pro²⁸ substitution may provide another set of internal deletion mutants.

The discovery that the activity of hIL-3 is retained in the disclosed double mutants or for any other mutant lacking large parts of the protein (e.g. approximately 25 percent) has another potential advantage. A pharmaceutical composition containing hIL-3 can be administered parenterally, intravenously or subcutaneously. The use of a hydrogel composed of biodegradable polymer enclosing the polypeptide and continuously releasing said polypeptide is limited by the amount of peptide that can be enclosed. Using a deletion mutant of the polypeptide with higher specific activity implies that on a molar basis more of the active substance can be enclosed in the same volume thereby increasing the time between successive administrations or possibly avoiding repeated administrations.

Deletion mutants can be used to alter the glycosylation sites. Thus enabling the production of glycosylated or unglycosylated polypeptides in eukaryotic host cells at will.

The mutants described above were used in epitope mapping experiments in order to determine where exposed segments of the molecule, which may be relevant in receptor binding, are located. To achieve this, monoclonal antibodies were prepared against mature hIL-3. Subsequently, these antibodies were used in western blotting experiments against mutant proteins. At least one antigenic fragment could be detected.

The new hIL-3 mutants according to the present invention are conveniently prepared by site-directed mutagenesis, but a variety of other techniques which are known in the art to cause modified proteins can also be used.

Suitable vectors which are capable of transforming microorganisms, which can express the mutated DNA sequences encoding the desired hIL-3 mutants, include expression vectors comprising said mutated sequences derived from the mature hIL-3 coding sequence, joined to expression regulating regions and terminator regions. These regions are chosen depending on the prokaryotic or eukaryotic cells which are used as host cells. Preferably, E. coli or Bacillus are used as host strains. However, fungi, yeast cells and tissue culture cells can also be employed (see EP-A-0275598).

Expression vectors for E. coli include pGB/IL-336 and pGB/IL-339, each containing the lacZ promoter, and for Bacillus pGB/IL-322, containing the α-amylase promoter and signal peptide, and derivatives thereof. Other suitable vectors for expression in Bacillus are, for example, HpaII promoter containing vectors and derivatives thereof. These and other vectors are all described in EP-A-0275598 and EPA 90200624.6 (filed March 15, 1990, based on the following priorities: EPA 89200660.2 filed on March 15, 1989 and EPA 89201967.0 filed on July 25, 1989).

For convenience it may be useful to use expression constructs which are capable of secreting the protein out of the host cell. This is for example done in the Bacillus constructs used. Isolation from inclusion bodies, as exemplified here in E. coli, is also possible.

Purification of the polypeptide obtained after expression is dependent on the host cell and the expression construct that is used. Generally the purification of hIL-3 muteins can be performed in the same way as the purification of native hIL-3. To obtain highly purified hIL-3 muteins the following steps are to be used; hydrophobic interaction chromatography, followed by anion exchange chromatography and optionally followed by gel filtration. It may also be sufficient to use only one or two of these purification steps (a detailed description of the purification of hIL-3 is disclosed in EPA 90200624.6 filed March 15, 1990, based on the following priorities: EPA 89200660.2 filed on March 15, 1989 and EPA 89201967.0 filed on July 25, 1989).

Briefly, after expression in E. coli as a first step the inclusion bodies containing the protein were isolated. After sonification in 8 M urea the protein was further purified by applying anion exchange chromatography. After removal of the urea, filter sterilization yielded a protein that was used for subsequent biological and biochemical characterization. The use of Bacillus as a host strain, in combination with secretion of the polypeptides, yielded highly pure polypeptides after the combination of hydrophobic interaction and anion exchange chromatography. Furthermore, it is obvious that mutants having altered charges may exhibit different physical properties, which requires special elution conditions or other column materials than the ones specifically used in the Examples.

The purified muteins can be formulated into pharmaceutical compositions by admixture with a pharmaceutically acceptable nontoxic carrier. As mentioned above, such compositions may be prepared for use for parenteral (subcutaneous, intramuscular or intravenous) administration particularly in the form of liquid solutions or suspensions. The compositions may conveniently be administered in unit dosage form and may be prepared by any of the methods known in the pharmaceutical art, for example, as described in Remington's Pharmaceutical Sciences, Mack Publishing Company, Easton, Pa., 1970. Formulations for parenteral administration may contain as common excipients sterile water or saline, polyalkylene glycols such as polyethylene glycol, oils of vegetable origin, hydrogenated naphtalenes and the like.

The materials of this invention can be employed as the sole active agent in a pharmaceutical or can be used in combination with other active ingredients. Other active ingredients can be selected for example from appropriate hematopoietins, CSFs and interleukins. These include GM-CSF, CSF-1, G-CSF, M-CSF, erythropoietin, IL-1α, IL-1β, IL-2, IL-4 to IL-8 and tumour necrosis factor.

A number of tests are available for assaying the biological activity and potency of hIL-3 analogs of the invention. For example, the analog can be tested using techniques well known in the art, to see if the protein stimulates colony formation by human hematopoietic progenitor cells. The colonies formed include erythroids, granulocytes and granulocyte macrophages. Alternatively, the ability of the subject analogs to stimulate DNA synthesis by human acute myelogenous leukemia (AML) blasts, as evidenced for example by labeled thymidine uptake, can be used to indicate activity.

The biologically active polypeptide analogs of human IL-3 as provided by the present invention can be used for both therapeutic and diagnostic purposes. Therapeutic uses include the treatment and prevention of malignant and non-malignant disorders. Due to the special properties of the presented mutants application can be improved.
Applications include treatment of:
- cytopenias and/or immunosuppression due to infections,
- cytopenias due to chemotherapy and/or irradiation,
- bone disorders such as bone fractures and osteoporosis,
- immunodeficiencies due to general anaesthetic procedures,
- recovery following bone marrow transplantation,
- adjunct to vaccination and adjunctive therapy of infections.

The invention is further illustrated by the following non-limiting examples.

### EXPERIMENTAL

### EXAMPLE 1

### Construction of Expression Vectors

### Construction of pGB/IL-336

In order to produce a polypeptide closely resembling the mature human IL-3, a construct was made lacking the 5' nontranslated and signal peptide encoding sequences. The IL-3 cDNA insert of pLH1 (see EP-A-0275598) was excised with HincII and HindIII, ligated to a synthetic oligonucleotide (SalI-overhang/blunt, 419/420, Table 1) comprising the sequence encoding the N-terminal 14 amino acids of the mature IL-3 and inserted into SalI-HindIII digested pTZ18R (Pharmacia). Following verification of the sequence, the complete insert was transferred to pUC8 for protein production (as described previously in EP-A-0275598), resulting in plasmid pGB/IL-335. To allow for direct sequencing and mutagenesis, the PvuI fragment of plasmid pTZ18R carrying the f1-origin of replication was used to replace the corresponding PvuI fragment of pUC8. The resulting bacterial expression plasmid pGB/IL-336 gives rise to a fusion protein of 145 amino acids with a calculated Mw of 16,384 dalton (Figure 1).

### Construction of pGB/IL-339

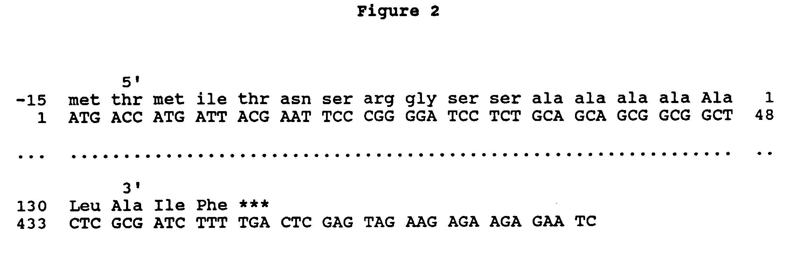

An alternative expression plasmid was constructed to facilitate efficient transfer of potentially interesting mutants into Bacillus expression vectors (see below). For this purpose pGB/IL-336 was digested with HpaI and HindIII to remove most of the 3'-terminal part of the IL-3 cDNA. The protruding ends were made blunt and subsequently a blunt fragment carrying the corresponding IL-3 sequences (nt 137-497) and the B. licheniformis alpha-amylase terminator from plasmid pGB/IL-337 was ligated into it, resulting in plasmid GB/IL-338. pGB/IL-337 had been constructed from pGB/IL-324 (as disclosed in the European Patent Application No. 892019670 which is incorporated herein by reference) by looping-out of 3' non-coding IL-3 cDNA and alpha-amylase structural sequences between the IL-3 stopcodon in the cDNA and the alpha-amylase terminator, using a synthetic oligonucleotide (Table 1, 944). This oligonucleotide also introduced a XhoI site just downstream of the IL-3 stopcodon. Plasmid pGB/IL-338 was digested with BamHI and HincII and part of the polylinker and 5'-terminal IL-3 sequences were substituted by the phosphorylated synthetic oligonucleotides 1055/1056 (Table 1) giving rise to plasmid pGB/IL-339. Thus the IL-3 gene was reconstructed with a slightly modified heterologous signal peptide encoding sequence. Now a fusion protein of 148 aa with calculated Mw of 16,541 dalton is synthesized (Figure 2). The DNA sequence of pGB/IL-339 was verified and found to be correct. The biological activity of the expressed fusion protein was found to be unaltered.

Another E. coli expression vector pGB/IL-340 was constructed, in which the lacZ promoter is followed immediately by sequences encoding the Bacillus alpha-amylase signal peptide precisely fused to mature IL-3, with the amylase terminator downstream thereof. This plasmid, which also serves as an intermediate for the construction of Bacillus expression vectors, was constructed by introduction of the NdeI-KpnI fragment from pGB/IL-337, carrying all the sequences just mentioned, into the NdeI-KpnI cleaved E. coli vector pTZ18RN. pTZ18RN is pTZ18R (Pharmacia) modified just upstream of the lacZ ATG startcodon to introduce an NdeI site, using oligonucleotide 731 (see Table 1). pGB/IL-340 gives rise to high production of IL-3 in E. coli.

### EXAMPLE 2

### Construction of Deletion and Substitution Mutants

In vitro mutagenesis was performed using synthetic oligonucleotides (Tables 1 and 2) according to the procedure developed by Kunkel et al. (1987) Meth. Enzymol. 154 367-382. Single stranded template DNA was prepared by transformation of pGB/IL-336 or pGB/IL-339 DNA into E.coli strain CJ236 (BioRad) and superinfection with M13K07 (Pharmacia) helper phage. Phage DNA was prepared according to standard procedures and fractionated on 0.6% low melting agarose to remove the helper phage ssDNA. The excised band was melted and extracted with phenol. pGB/IL-336 or pGB/IL-339 ssDNA was recovered by butanol concentration and ethanol precipitation and tested for primer-dependent DNA synthesis in a sequencing reaction.

Purified pGB/IL-336 or pGB/IL-339 ssDNA (± 100 ng) was annealed with 20 ng of phosphorylated primer at 65°C and slowly cooled to room temperature. Synthesis of the complementary strand was performed using 1-2 µg of gene 32 protein, 4 units of T4-DNA polymerase and 2.5 units of T4-DNA ligase. Reaction was carried out at 0°C for 5 min, at 20°C for 10 min and at 37°C for 90 min. After completion, one third of the reaction mixture was mixed with thawed competent (Hanahan, D. (1985) in: "DNA Cloning" (D.M. Glover ed.) Vol. 1, IRL Press, Washington) JM109 cells and plated. Individually picked colonies were used to inoculate liquid media and the cells were superinfected with the M13K07 helper phage to produce ssDNA. The sequence of the clones was verified using the M13 reverse sequencing primer and two IL-3 sequence specific primers (Table 1; 823: nt 118-137 and 824: nt 261-280).

The double cysteine mutant (9045) was constructed by ligation of the corresponding BstBI-BamHI fragments of the plasmids carrying the mutant IL-3 genes 904 and 905. Similarly, the combination mutants X and Z were constructed by ligation of fragments containing a 5' deletion and fragments containing a 3' deletions. Resulting clones were checked by restriction enzyme analysis and sequence analysis.

Mutant 9329 was derived from 939 following HpaI and BamHI digestion and ligation with oligonucleotides 1424/1425. Clones were verified first for loss of the HpaI site and subsequently for correct DNA sequence.

From all transformants, plasmid DNA was isolated for a second round of transformation into JM109 cells to exclude potential contamination with the parental IL-3 construct. Following sequence verification, these clones were used for protein production.

Some of the mutants described here (811, 933 and 9329) were introduced into pGB/IL-340. This was done by ligating the PstI-XhoI fragment of a pGB/IL-339 derived mutant into PstI-XhoI cleaved pGB/IL-340, this resulted in plasmids pGB/IL-340/811, pGB/IL-340/933 and pGB/IL-340/9329.

Bacillus expression vectors for IL-3 muteins were constructed by exchanging the PstI-HindIII fragment of pGB/IL-322 (carrying part of the alpha-amylase signal peptide, the complete mature IL-3 cDNA sequence and the amylase terminator) with the PstI-HindIII fragment of a pGB/IL-340 mutant derivative (carrying part of the alpha-amylase signal peptide, the mutant IL-3 cDNA sequence and the alpha-amylase terminator). The Bacillus expression vectors for the muteins 811, 933 and 9329 were named pGB/IL-322/811, pGB/IL-322/933 and pGB/IL-322/9329 respectively.

Another mutant (1455 lacking amino acids 130-133) was constructed directly in pGB/IL-340 by loop-out mutagenesis, using oligonucleotide 1455 (Table 1), resulting in plasmid pGB/IL-340/1455. Subsequently, the mutant sequence was transferred to pGB/IL-322 in a similar way as described for mutants 811, 933 and 9329 resulting in plasmid pGB/IL-341.

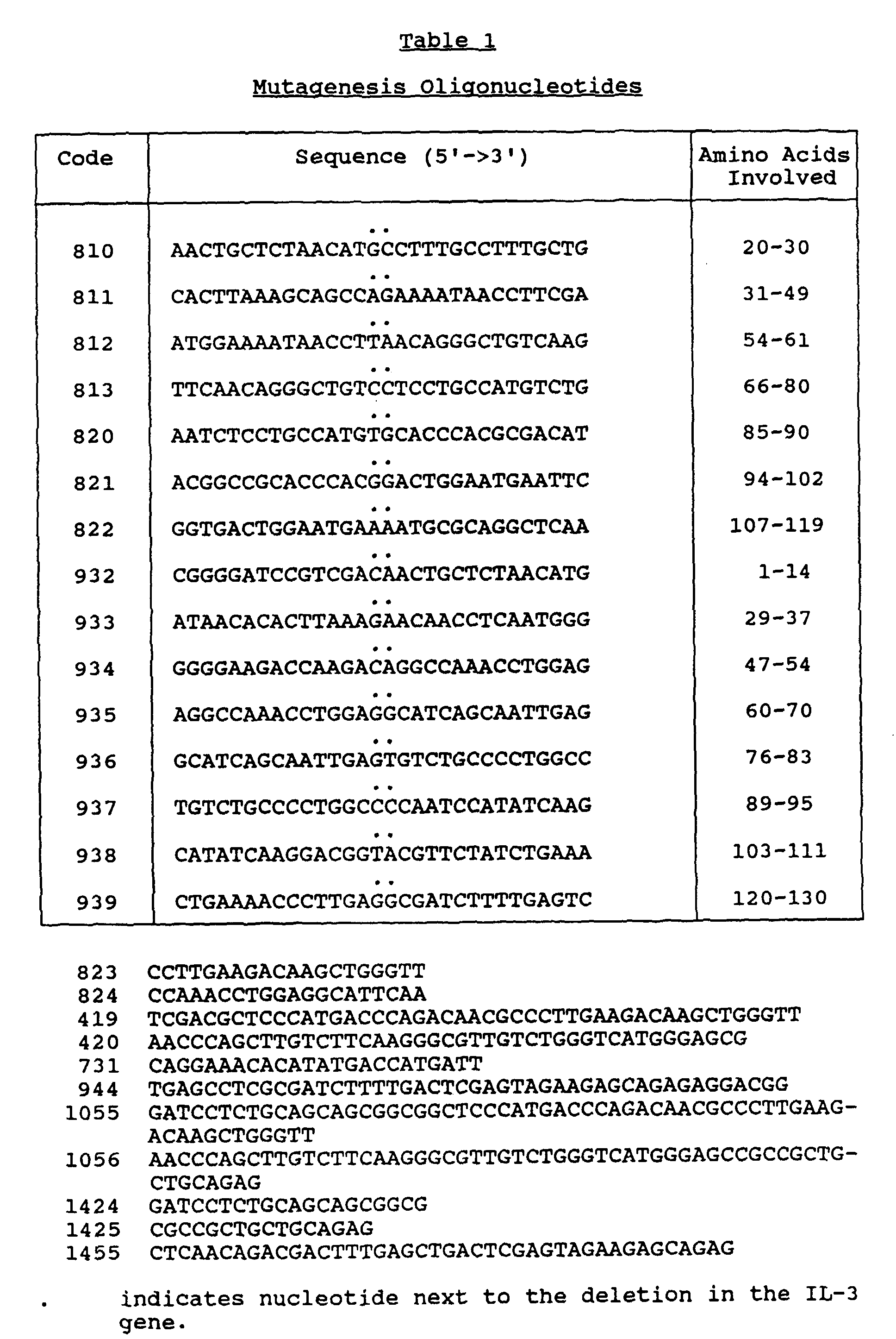

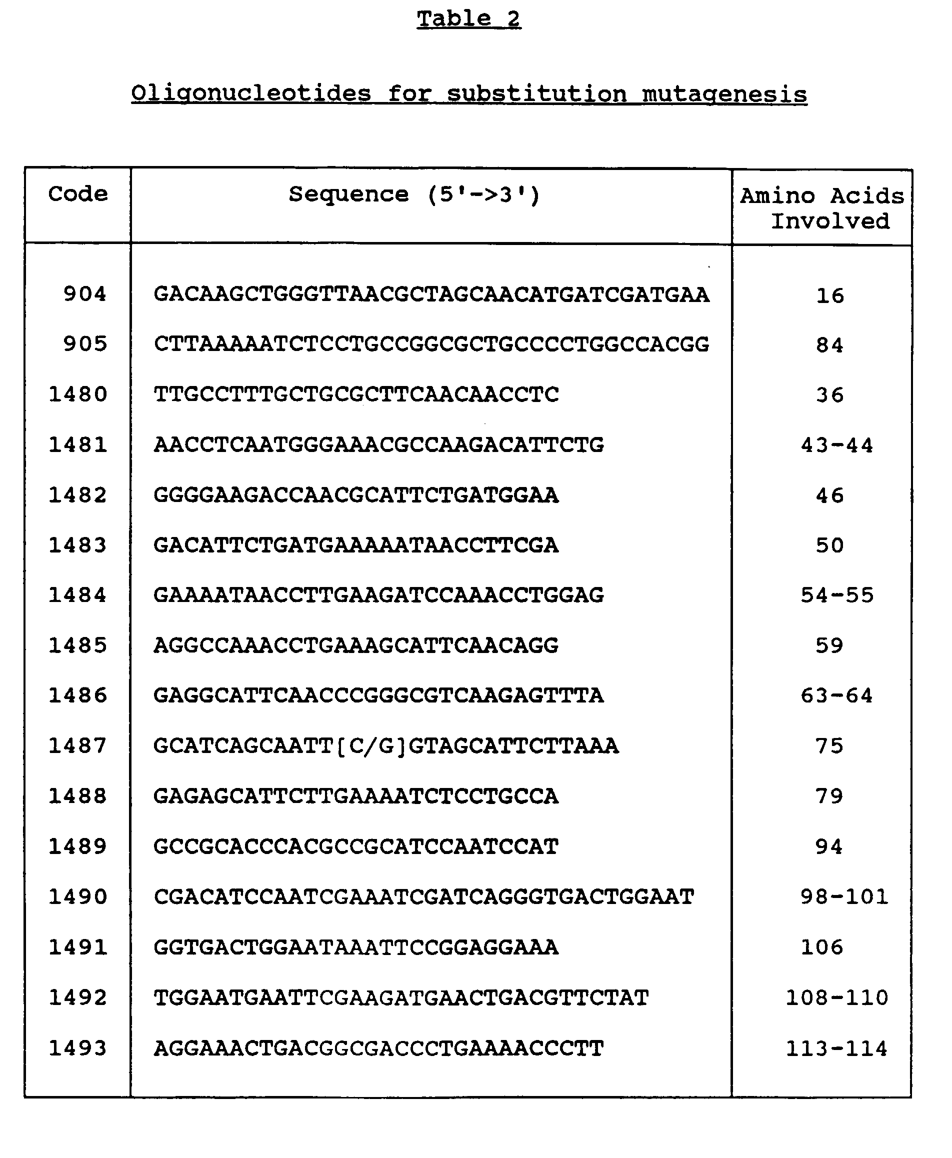

### EXAMPLE 3

### Purification of IL-3 Muteins from E. coli or from Bacillus

E. coli JM109 cultures (100 ml) were inoculated with 0.5 ml of a fresh overnight culture of the (mutant) IL-3 clone and grown at 37°C until an OD of 0.4-0.6 at 550 nm was reached. Plasmid directed protein synthesis was induced by addition of 1 mM of IPTG (Pharmacia). After an additional culture for 3-16 hrs, the cells were collected by centrifugation (10 min, 4000 rpm at 4°C) and stored frozen.

The bacteria were suspended in 10 ml of TE (10 mM Tris-HCl pH 8; 1 mM EDTA) and lysozyme was added (0.025% = 500 µg/ml). Following incubation for 30 min at room temperature, MgCl₂ and DNase were added to final concentrations of 10 mM and 20 µg/ml, respectively. After incubation at 37°C for 15 min, Tween 20 (0.2%), DTT (2 mM) and PMSF (0.1 mM) were added. The suspension was cooled on ice and vigorously sonified (two times 35 sec). The homogenate was clarified by centrifugation (30 min 15,000xg, 10,000rpm in a Beckman JS13.1 rotor) at 4°C and the supernatant was discarded.

The pellet was resuspended in 4 ml of 55% sucrose in buffer TPD (50 mM Tris-HCl, pH 8; 0.1mM PMSF and 2mM DTT) by sonification and either pelleted and solubilized in urea for direct assaying of biological activity or layered onto a discontinuous sucrose gradient (2 ml portions of 75% and 60% of sucrose in TPD buffer), essentially as described in WO 88/00598. Following centrifugation at 200,000xg (35,000 rpm in a Sorvall TH641 rotor) at 25°C for 2 hours, the inclusion bodies containing the IL-3 proteins were recovered from the 75% sucrose interphase.

After at least 4-fold dilution, the inclusion bodies were pelleted at 25,000xg (30 min at 13,000 rpm in the Beckman JS13.1 rotor) and sonificated in 5ml of 8M urea containing 50 mM Tris-HCl pH 8.9 and 2 mM DTT and left overnight at 4°C. The clarified solution was subsequently applied to a 3 ml DEAE-Sepharose Fast Flow column (Pharmacia), equilibrated with 8 M urea, 50 mM Tris-HCl pH 8.9 and 1 mM DTT buffer. The IL-3 protein was bound to the column and step eluted with 75 mM NaCl in the same buffer. The eluted protein was dialyzed against several portions of 10 mM Tris-HCl pH 8.0 and 1 mM DTT buffer and made isotonic by adding 10-fold concentrated RPMI cell culture medium (Sigma) containing 1% bovine serum albumin. The filter sterilized (0.22 µm Millex-GV, Millipore) solution was used for biochemical and biological characterization.

Mutant proteins 811, 933, 1455, 9329, X and Z produced by Bacillus were exported into the culture medium and did not require such drastic purification procedures for testing of biological activity. Therefore the clarified supernatant (1 litre) (adjusted to 5mM EDTA, 1mM PMSF and 1 M ammonium sulphate) was passed over a 15-20 ml Fractogel TSK Butyl 650 (C) column (Merck) equilibrated with 1 M ammonium sulphate in 10 mM Tris-HCl pH 7.0 buffer. The bound IL-3 protein was eluted with 10 mM Tris-HCl buffer and subsequently passed over a 1.5 ml DEAE-Sepharose Fast Flow colum equilibrated with 10 mM Tris-HCl pH 8.0 buffer. The flow through was collected and adjusted to 70% ammonium sulphate to concentrate the IL-3 protein. The precipitate was collected by centrifugation at 10,000 rpm (JS-13 rotor), dissolved and dialyzed against 10 mM Tris-HCl pH 8.0, 1 mM DTT buffer.

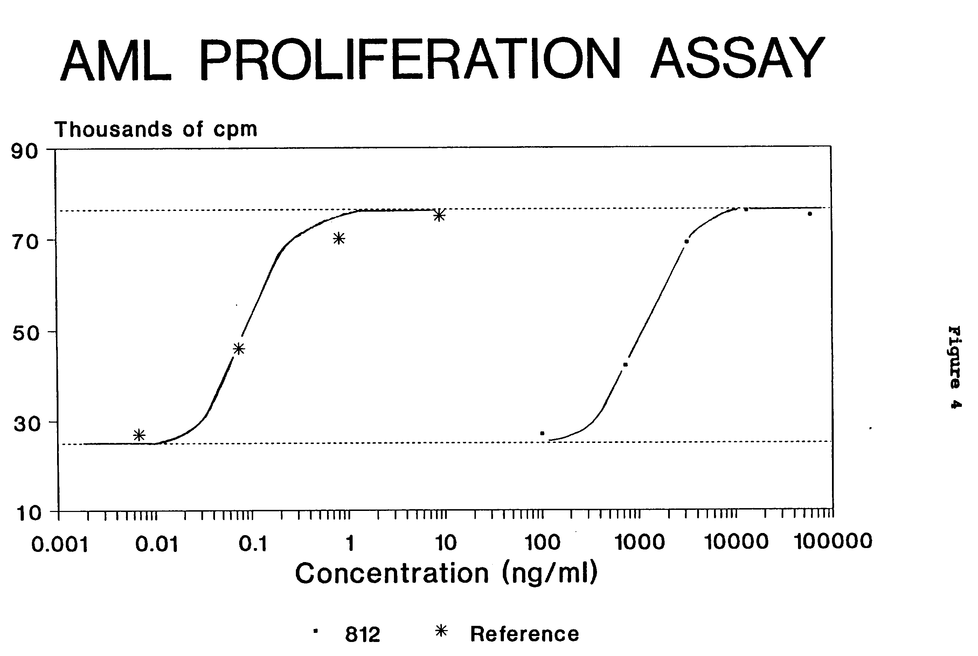

Samples corresponding to 25-100 µl of the original bacterial culture were analyzed on 0.75x75x100 mm (BioRad miniprotean II) 13.5% SDS-polyacrylamide gels (acryl/bisacryl=29/1). Proteins were visualized by either Coomassie Brilliant Blue G250 staining or immunological methods. Figure 4 shows an example of purified muteins expressed in E. coli.

The expression in E. coli of mutant pGB/IL-339 derived IL-3 proteins was generally higher than the same mutant protein from the pGB/IL-336 expression vector. The applied purification procedure was not devised to render completely pure IL-3 protein preparations. In general, minor higher molecular weight contaminants were observed on SDS gels. Using densitometric scanning of stained SDS gels, the amount of IL-3 protein was determined. Although occasionally severe loss of protein occurred following dialysis, total recovery was generally 0.1-1 mg of "purified" IL-3 protein. The only exception were the mutants 811 and 933, which gave no fusion protein production in either of the E. coli expression vectors. Northern analysis indicated a strong reduction of IL-3 specific RNA in the bacteria, whereas no significant difference in the plasmid DNA content was observed. This result suggests that probably mRNA stability is decreased through the deletions introduced in the eukaryotic IL-3 cDNA sequence.

The only convenient method for synthesis of the muteins 811 and 933 is expression in Bacillus licheniformis. Although the amount of protein produced is very low, compared to the amount of protein produced by Bacillus strains synthesizing other muteins such as 9329 and 1455, enough is made to partially purify the muteins 811 and 933 in sufficient quantities to carry out the biological assays described herein.

### EXAMPLE 4

### Immunological characterization of the IL-3 Muteins

Preparation of polyclonal and monoclonal antisera has been described in WO 88/04691.

For immunological detection of IL-3 muteins, the gel fractionated proteins (see Example 3) were transferred onto nitrocellulose (0.2 µm, Schleicher and Schuell BA83) using the semi-dry blotting system (Novablot, Pharmacia/LKB) with a continuous buffer system (39 mM glycine, 48 mM Tris, 0.0375% SDS and 20% methanol) at 1.2 mA/cm² for 90 min. The nitrocellulose filter was subsequently air dried, preincubated with a 3% bovine serum albumin (BSA, Sigma) solution (10 mM Tris-HCl pH 7.6; 350 mM NaCl; 0.1% PMSF and 0.1% sodium azide) and incubated for 4-16 hr with the polyclonal (10⁻³ dilution) or monoclonal (depending on the intended aim of the experiments) antibodies directed against human IL-3 (MCA A1, A4, A5, A8, A18 at 10⁻⁴ dilution) in RIA buffer (10 mM Tris/HCl, pH 7.6; 150 mM NaCl; 1% Triton X-100; 0.1% SDS; 0.5% Na-deoxycholaat; 0.1 mM PMSF and 0.3% BSA). Immunological complexes were visualized using biotinylated anti-rabbit or mouse Ig, streptavidin-biotinylated horseradish peroxidase (Amersham International, Amersham, UK) and 4-chloro-1-naphtol (Gibco-BRL) according to the protocols provided by the manufacturer. Alternatively, antiserum incubations were performed in Tris-buffered saline plus 0.05% Tween 20. Alkaline phosphatase-linked anti-mouse Ig complexes were visualized according to standard protocols (Promega).
The data, pertaining to the interaction of specific monoclonal antibodies with hIL-3 muteins in western blotting experiments, are presented in Table 3.

**Table 3**

| Evaluation of IL-3/MCA data | | | | | | | | |
|---|---|---|---|---|---|---|---|---|
| MUTANT | 4810 | 933 | 811 | 934 | 812 | 935 | 4813 | ALL OTHER |
| (AA) | 20-30 | 29-37 | 31-49 | 47-54 | 54-61 | 60-70 | 66-80 | |
| MCA | | | | | | | | |
| A1 | ++ | -- | -- | ++ | ++ | ++ | ++ | ++ |
| A4 | ++ | ++ | ++ | ++ | + | + | + | ++ |
| A5 | ++ | ++ | -- | -- | + | ++ | ++ | ++ |
| A8 | ++ | ++ | -- | ++ | ++ | ++ | ++ | ++ |
| A18 | ++ | -- | -- | ++ | ++ | ++ | ++ | ++ |
| 2 | ++ | -- | -- | ++ | ++ | ++ | ++ | ++ |
| 3 | ++ | ++ | -- | -- | + | ++ | ++ | ++ |
| 6 | ++ | ++ | -- | + | ++ | ++ | ++ | ++ |
| 10 | ++ | ++ | -- | + | ++ | ++ | ++ | ++ |
| 12 | ++ | -- | -- | ++ | ++ | ++ | ++ | ++ |
| 13 | + | -- | -- | ++ | ++ | + | + | ++ |
| 14 | ++ | ++ | -- | -- | + | ++ | ++ | ++ |
| 15 | ++ | -- | -- | ++ | ++ | ++ | ++ | ++ |
| 26 | ++ | ++ | -- | + | ++ | ++ | ++ | ++ |
| 27 | ++ | ++ | ++ | ++ | + | + | + | ++ |
| 32 | ++ | -- | -- | ++ | ++ | ++ | ++ | ++ |
| 33 | ++ | -- | -- | ++ | ++ | ++ | ++ | ++ |

| | 36D->R | 43ED->KR | 46D->R | | | | | |
|---|---|---|---|---|---|---|---|---|
| | 1480 | 1481 | 1482 | | | | | |
| A1 | ++ | ++ | ++ | | | | | |
| A4 | ++ | ++ | ++ | | | | | |
| A5 | ++ | ++ | -- | | | | | |
| A8 | ++ | -- | + | | | | | |
| A18 | + | ++ | ++ | | | | | |
| A : ascites monoclonals. | | | | | | | | |
| ++: full response on western blot. | | | | | | | | |
| + : at least 5-fold reduced response. | | | | | | | | |
| --: no reaction observed. | | | | | | | | |

From analysis of the results presented in Table 3 it is evident that the majority of the monoclonal antibodies raised against the gel-purified denatured fusion protein, are directed against epitopes located between residues 30 and 50 of the mature IL-3 polypeptide. These epitopes mostly reflect linear polypeptide chains, only one example (13) of a discontinuous epitope has been identified. The largest group is capable of identifying the 933 mutant, but fails to react with the 811 mutant. Thus, the epitope must reside between residues 37 and 50. This region turns out to be highly hydrophilic with a postulated β-turn and α-helix (DNASIS software) and was proposed to be an antigenic determinant using Hopp and Wood algorithm. The second group of antibodies is characterized by the absence of reaction with mutant 933 (which lacks 9 amino acids). This hydrophobic proline-rich, coil region is not predicted to be a major antigenic site.

More refined localization of these epitopes comes from reaction of the ascites monoclonals with the following substitution mutants: Asp³⁶ -> Arg, Glu⁴³Asp -> Lys⁴³Arg and Asp⁴⁶ -> Arg.

Further characterization of the ascites monoclonals by double sandwich ELISA revealed lack of interference between A1 and A5 or A8, and between A5 and A18. Competition ELISA showed cross-competition between A1 and A18, between A5 and A8, and between A8 and A18. No cross-competition was observed with A4. These data are in agreement with the immunoblotting experiments. Furthermore, ascites antibodies were found to react with hIL-3 preparations from mammalian, bacterial and yeast cultures, irrespective of glycosylation state.

From the combined information of these immunological tests epitopes can be localized as follows:
- A1 and A18 are aimed against an epitope localized between amino acids 29 and 37. Furthermore from the 36 D -> R mutant it can be concluded that the A18 epitope is localized somewhat more towards the C-terminus than the A1 epitope.
- A5 is aimed against an epitope localized between amino acids 45 and 54.
- A8 is aimed against an epitope localized between amino acids 36 and 47.

### EXAMPLE 5

### Biological characterization of the IL-3 Muteins

AML DNA synthesis was tested on human AML193 cells. Human AML193 cells (ATCC: CRL-9589) were maintained in Serum Free Medium (SFM), [Iscove's Modified Dulbecco's Medium (Gibco BRL) containing 0.1% BSA (Behringwerke AG, Marburg), 10 µg/ml of Insulin (Organon) and Transferrin, 0.1 mM β-mercaptoethanol] and 10 to 20 units of human IL-3. Dilutions of (mutant) IL-3 preparations were prepared (50 µl) in round bottom 96 wells plates in SFM. Washed cells (1-2x10⁴ in 50 µl SFM) were added and cultured for 6 days. ³H-Thymidine (0.1 µCi, 2 µCi/mmol) was added in 20 µl SFM and cells were harvested 16 hours later. An unit of IL-3 activity was defined as the amount required to give 50% of maximal DNA synthesis in this assay. For the reference IL-3 preparation the specific activity varied between 2 x 10⁶ to 2 x 10⁷ Units per mg of protein. In general, the biological activity of the muteins was compared to this reference preparation.

Figure 4 shows an example of the AML proliferation assay in which mutein 812 is compared with the reference IL-3 expressed in Bacillus. The graph shows that the mutein 812 has an activity which is reduced by about 4 orders of magnitude with respect to unmodified IL-3. However, the mutein is still able to stimulate the AML cells to maximal activity, only more protein is needed.

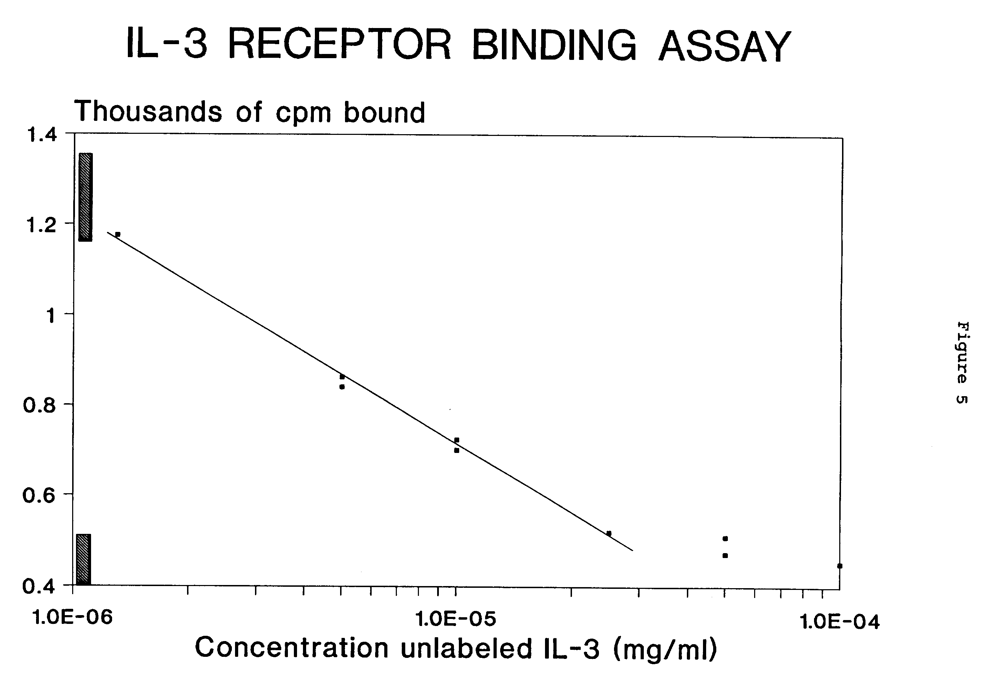

An IL-3 receptor binding assay was performed with purified recombinant hIL-3 (see EP-A-0275598 and EPA 90200624.6). IL-3 was radiolabeled with the Bolton-Hunter reagent (as described by Budel et al. Blood, 74 (1989) 565-571). The specific activity was estimated at 80,000 cpm/ng of IL-3. As target, either donor leukocytes, patient AML cells, or MV4-11 (ATC: CRL-9591) cells were used. Cells were washed in Hanks Balanced Salt solution and suspended in Alpha-MEM plus 1% BSA. Usually, 3-10x10⁶ cells were incubated with 200-300 pM of radiolabeled IL-3 and various concentrations of unlabeled mutant protein in a total volume of 200 µl for 1hr at 37°C. Cell-bound labeled IL-3 was centrifuged for 10 min at 1000xg at 4°C through a 0.5 ml cushion of bovine calf serum in an Eppendorf tube (Budel et al., 1989). The tubes were frozen in liquid nitrogen and the tips cut off for counting in a Packard gamma counter. Competition binding was determined relative to the reference preparation. Figure 5 shows an example of an Il-3 receptor binding experiment. Radioloabeled IL-3 binding to patient AML blasts is competed with unlabeled IL-3 protein (reference preparation). Due to the low number of receptors (less than 200) on the target cells, data for competition mostly varied by one order of magnitude.

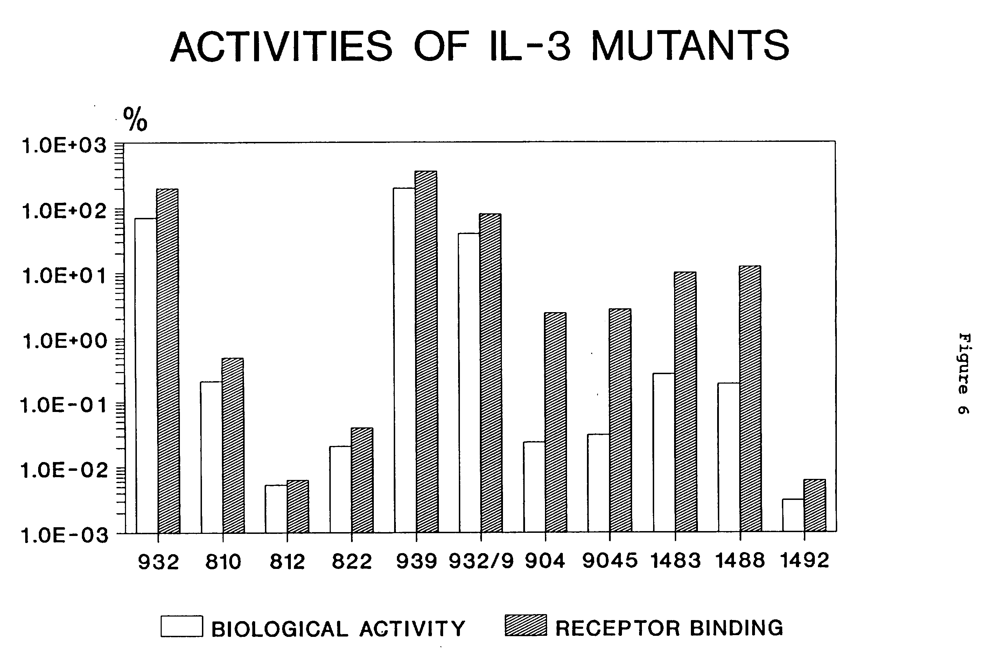

The results from the above assays concerning the specified deletion and substitution mutants are presented in Table 4. Part of the results are also presented graphically in Figure 6.

As can be seen IL-3 fusion proteins derived from both pGB/IL-336 and pGB/IL-339 expression plasmids were identical in biological activity and were not significantly different from the B. licheniformis reference preparation derived from expression of the full length IL-3 cDNA in pGB/IL-322 (see EPA 89201967.0). Apparently, no negative effect on the biological activity is exerted by the heterologous leader peptide in the E. coli fusion proteins. Most IL-3 deletion mutants were significantly reduced in biological activity. Deletions between amino acids 50 and 105, resulted in more than 10,000-fold reduction in specific activity. However, in all cases residual biological activity was detected, indicating the sensitivity of the bio-assay and the absence of toxic components in the protein preparations. Complete recovery of biological activity was observed for deletion mutants 932 (1-14) and 939 (120-130) and the corresponding double mutant 9329 as well as for the C-terminal mutant 1455. The deleted N- and C-terminal sequences are apparently not involved in binding to the receptor or proper folding of the molecule. In contrast, single substitutions of the cysteine residues (mutants 904 and 905) result in a 5000-fold reduction of biological activity. Similar effects were seen with a double cysteine mutant (9045), indicating that the S-S bridge plays an important stabilizing role in the native IL-3 molecule.

The muteins expressed in E. coli described in the section above are all fusion proteins. To provide evidence that the extra N-terminal amino acids are not necessary as compensation for the loss of the deleted IL-3 specific amino acids, construct pGB/IL-322/9329 was made and expressed in B. licheniformis. This construct gives rise to a 1-14/ 120-130 IL-3 mutein without any extra amino acids at the N-terminus. The activity of this mutein is comparable to the wild-type molecule proving that only the residual 108 amino acids are responsible for the IL-3 activity.

Receptor binding of most of the mutant IL-3 proteins was determined. The binding experiments do not allow for detailed comparison of receptor binding activity due to the low receptor numbers on the target cells. However, in general there is a good correlation between biological activity and receptor binding (Table 4). For most mutant IL-3 proteins, the ratio between relative binding and biological activity is approximately 1. The muteins 1483, 1488, 904 and 9045 show considerably higher ratios (Table 4 and Figure 6), indicating potential antagonistic activity. This means that these molecules can be regarded as partial antagonists of IL-3. Upon further experimentation muteins may be found that have the same binding capacity as native IL-3 but that have a 10⁴-fold lower activity. Those molecules would be true antagonists of IL-3. The approach described in this invention provides the means and methods to obtain such molecules.

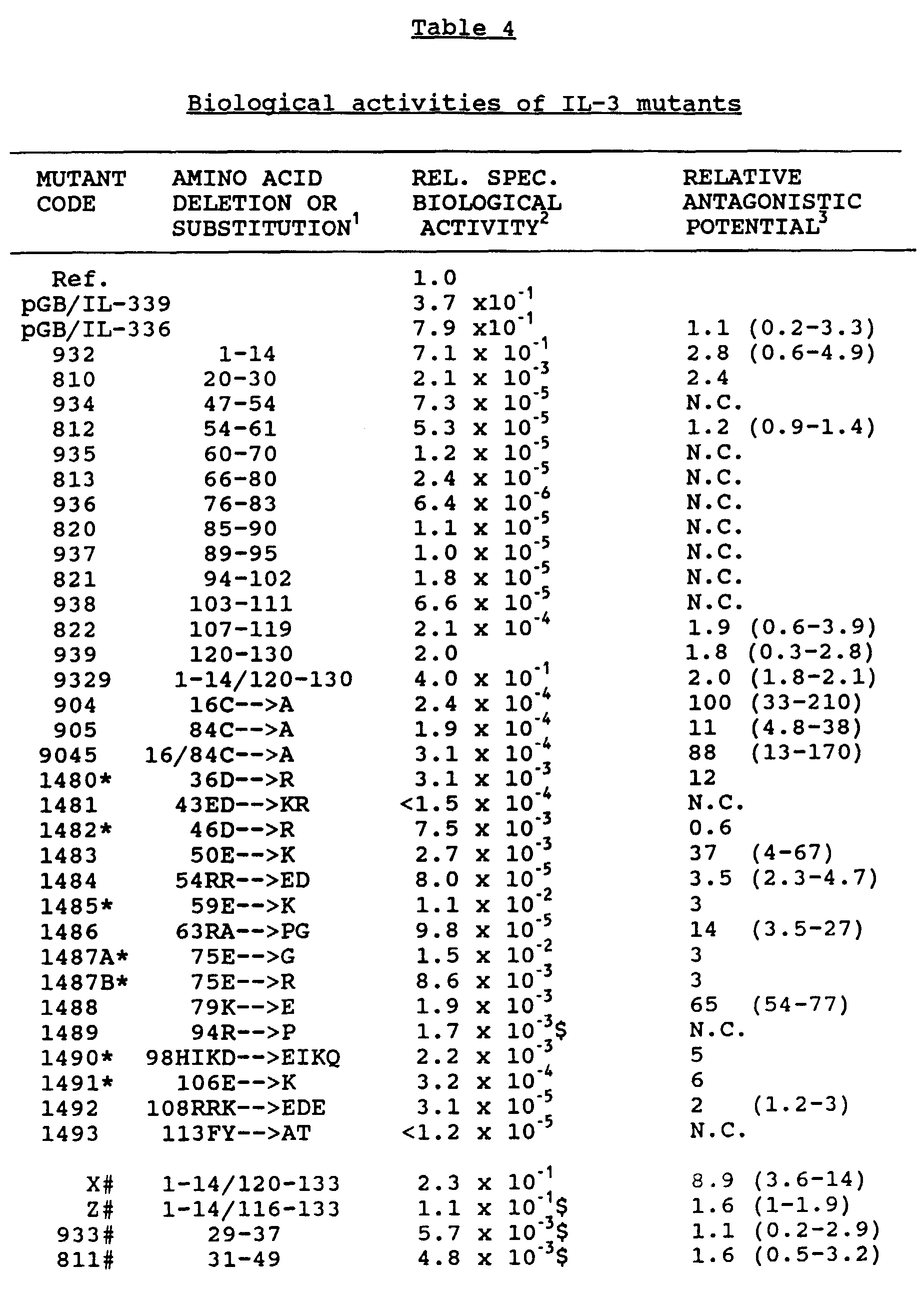

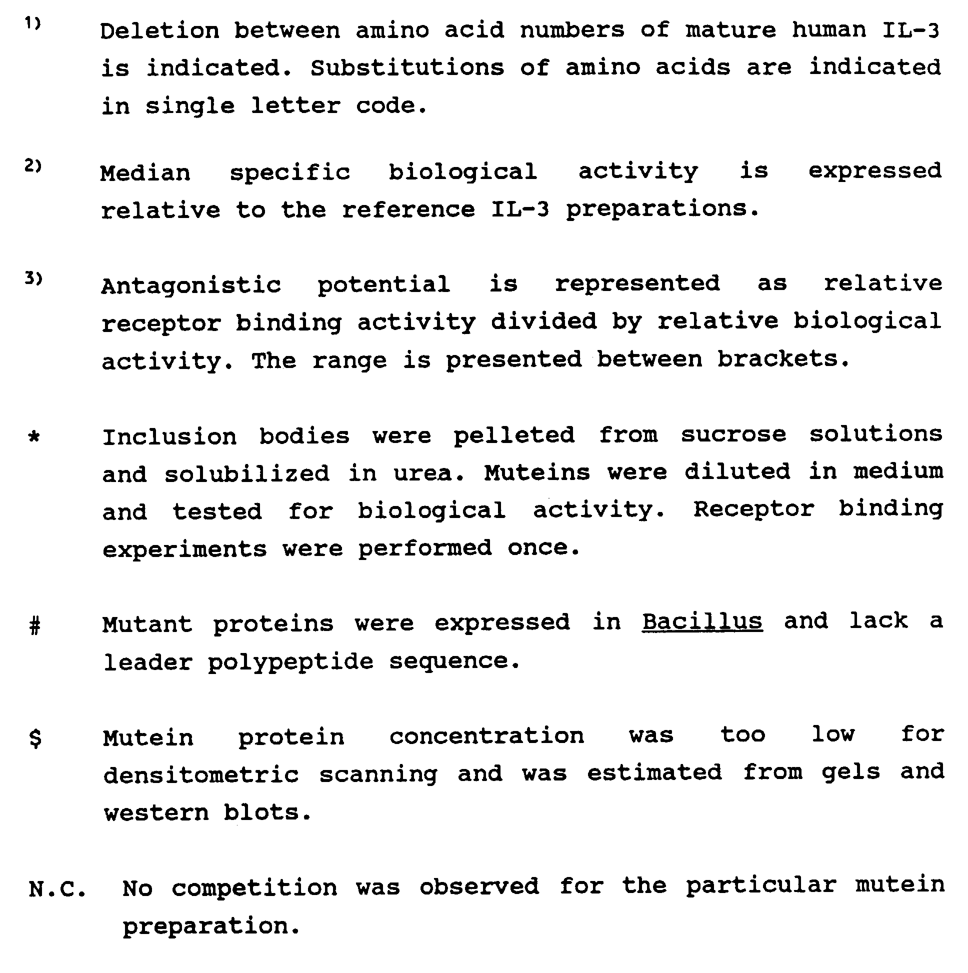

All publications (including patents and patent applications) mentioned in this specification are indicative to the level of skill of those skilled in the art to which this invention pertains. All publications are herein incorporated by reference to the same extent as if each individual publication was specifically and individually indicated to be incorporated by reference.

Although the foregoing invention has been described in some detail by way of illustration and example for purposes of clarity of understanding, it will be apparent to one of ordinary skill in the art that many changes and modifications can be made thereto without departing from the spirit or scope of the appended claims.

## Claims

1. A biologically active polypeptide analog of human IL-3 which is characterized in that it contains a deletion of amino acids 116-133, 120-130, 130-133 or 120-133.

2. A biologically active polypeptide analog of human IL-3 according to claim 1 which is further characterized in that it also contains a deletion of amino acids 1-14.

3. A biologically active polypeptide analog of human IL-3 which is characterized in that it contains a deletion of amino acids 1-14.

4. A pharmaceutical composition comprising, as an active ingredient, a biologically active polypeptide analog of human IL-3 as defined in any one of claims 1-3, or a derivative thereof, in conjunction with a pharmaceutically acceptable carrier.

5. A pharmaceutical composition according to claim 4, further comprising a pharmaceutically effective amount of a substance selected from the group consisting of GM-CSF, CSF-1, G-CSF, M-CSF, erythropoietin, IL-1α IL-1β, IL-2, IL-4 to IL-8 and tumour necrosis factor.

6. A DNA fragment containing a DNA sequence encoding a polypeptide according to any one of the claims 1-3.

7. A cloning vehicle containing a DNA sequence encoding any one of the polypeptides according to claims 1-3, in combination with transcription and translation regulation sequences that enable the expression of said polypeptides within suitable host cells.

8. A host cell transformed with a cloning vehicle according to claim 7.

9. A process for obtaining a DNA sequence encoding a biologically active polypeptide analog of human IL-3, according to one of the claims 1-3, comprising;
- isolating a DNA sequence encoding the mature hlL-3 protein,
- performing in vitro mutagenesis to delete desired nucleotides.

10. A process for obtaining a biologically active polypeptide analog of human IL-3, according to one of the claims 1-3, comprising;
- introducing the DNA sequence encoding said polypeptide in an expression vector, so that it is under the control of suitable expression regulating sequences,
- introducing said expression vector into a suitable host cell,
- culturing the host cells,
- recovering the protein produced by said host cells.

## Patentansprüche

1. Ein zu humanem IL-3 analoges, biologisch aktives Polypeptid, dadurch gekennzeichnet, daß es eine Deletion der Aminosäuren 116-133, 120-130, 130-133, oder 120-133 enthält.

2. Ein zu humanem IL-3 analoges, biologisch aktives Polypeptid gemäß Anspruch 1, weiter dadurch gekennzeichnet, daß es auch eine Deletion der Aminosäuren 1-14 enthält.

3. Ein zu humanem IL-3 analoges, biologisch aktives Polypeptid, dadurch gekennzeichnet, daß es eine Deletion der Aminosäuren 1-14 enthält.

4. Eine pharmazeutische Zubereitung, die als einen aktiven Bestandteil ein zu humanem IL-3 analoges, biologisch aktives Polypeptid nach einem der Ansprüche 1 - 3 oder ein Derivat davon, in Verbindung mit einem pharmazeutisch akzeptablen Träger, umfasst.

5. Eine pharmazeutische Zubereitung nach Anspruch 4, die weiterhin eine pharmazeutisch wirksame Menge einer Substanz umfasst, die ausgewählt ist aus der Gruppe umfassend GM-CSF, CSF-1, G-CSF, M-CSF, Erythropoietin, IL-1α, IL-1β, IL-2, IL-4 bis IL-8 und Tumor-NekroseFaktor.

6. Ein DNS-Fragment, das eine für ein Polypeptid gemäß einem der Ansprüche 1 - 3 kodierende DNS-Sequenz enthält.

7. Ein Klonierungsvehikel, das eine DNS-Sequenz, die für ein beliebiges der Polypeptide gemäß einem der Ansprüch 1 - 3 kodiert, in Kombination mit Transkriptions- und Translationsregulierungssequenzen, welche die Expression des Polypeptides in einer geeigneten Wirtszelle ermöglichen, enthält.

8. Eine Wirtszelle, die mit einem Klonierungsvehikel gemäß Anspruch 7 transformiert ist.

9. Ein Verfahren, um eine für ein zum humanen IL-3 analoges, biologisch aktives Polypeptid gemäß einem der Ansprüche 1 - 3 kodierende DNS-Sequenz zu erhalten, umfassend:
- Isolierung einer das reife hIL-3 Protein kodierenden DNS-Sequenz,
- Durchführung von in-vitro Mutagenese zur Entfernung gewünschter Nukleotide.

10. Ein Verfahren, um ein zum humanen IL-3 analoges, biologisch aktives Polypeptid gemäß einem der Ansprüche 1 - 3 zu erhalten, umfassend:
- Einführung der für das Polypeptid kodierenden DNS-Sequenz in einen Expressionsvektor, so daß sie unter der Kontrolle geeigneter, expressionsregulierender Sequenzen ist,
- Einführung des Expressionsvektors in eine geeignete Wirtszelle,
- Züchten der Wirtszellen,
- Rückgewinnen des von den Wirtszellen hergestellten Proteins.

## Revendications

1. Polypeptide biologiquement actif, analogue à 1'IL-3 humaine, caractérisé en ce qu'il comprend une délétion des acides aminés 116-133, 120-130, 130-133 ou 120-133.

2. Polypeptide biologiquement actif, analogue à 1'IL-3 humaine suivant la revendication 1, caractérisé en ce qu'il comprend également une délétion des acides aminés 1-14.

3. Polypeptide biologiquement actif, analogue à 1'IL-3 humaine, caractérisé en ce qu'il comprend une délétion des acides aminés 1-14.

4. Composition pharmaceutique comprenant, comme ingrédient actif, un polypeptide biologiquement actif, analogue à 1'IL-3 humaine, comme définis dans l'une quelconque des revendications 1 à 3, ou un de ses dérivés, associé à un vecteur pharmaceutiquement acceptable.

5. Composition pharmaceutique suivant la revendication 4, comprenant de plus, une quantité pharmaceutiquement efficace d'une substance sélectionnée parmi le groupe composé de GM-CSF, CSF-1, G-CSF, M-CSF, érythropoïétine, IL-1α, IL-1β, IL-2, IL-4 à IL-8, et facteur de nécrose tumorale.

6. Fragment d'ADN, contenant une séquence d'ADN codant pour un polypeptide selon l'une quelconque des revendications 1 à 3.

7. Véhicule de clonage, contenant une séquence d'ADN codant pour l'un des polypeptides selon l'une quelconque des revendications 1 à 3, combinée avec les séquences de transcription et de régulation de la traduction, qui rendent possible l'expression des polypeptides dans des cellules hôtes appropriées.

8. Cellule hôte transformée par un véhicule de clonage suivant la revendication 7.

9. Procédé de préparation d'une séquence d'ADN codant pour un polypeptide biologiquement actif, analogue à 1'IL-3 humaine suivant l'une quelconque des revendications 1 à 3, comprenant:
- l'isolement d'une séquence d'ADN codant pour la protéine hIL-3 mature;
- la réalisation de la mutagénèse in vitro pour déléter les nucléotides désirés.

10. Procédé de préparation d'un polypeptide biologiquement actif, analogue à l'IL-3 humaine suivant l'une quelconque des revendications 1 à 3, comprenant:
- l'introduction de la séquence d'ADN codant pour le polypeptide dans un vecteur d'expression, de telle sorte qu'il soit sous le contrôle de séquences appropriées de régulation de l'expression;
- l'introduction de ce vecteur d'expression dans une cellule hôte acceptable;
- la culture des cellules hôtes;
- la récupération de la protéine produite par ces cellules hôtes.
